# EUROPEAN PATENT APPLICATION

(11) **EP 4 809 983 A1**
(43) Date of publication of application: **23.09.2026**
(21) Application number: 24846693.0
(22) Date of filing: 14.11.2024
(51) Int. Cl.: A61F 2/16

(54) **INTRAOCULAR LENS FOR CATARACT SURGERY**

(30) Priority: 14.11.2023 ES 202330934
(71) Applicant: VOPTICA S.L., 30100 Murcia (ES)
(72) Inventor: GINIS, Harilaos, 30100 Murcia Murcia (ES); ARTAL SORIANO, Pablo, 30100 Murcia Murcia (ES)
(74) Representative: Elzaburu S.L.P.
(86) International application number: PCT/ES2024/070707
(87) International publication number: WO 2025/104362

(57) **Abstract**

Intraocular lens for cataract surgery, comprising an anterior surface and a posterior surface, such that they form a meniscus with the anterior surface being concave and the posterior surface being convex, wherein:
- the shape factor is between -1 and -2,
- the anterior surface has a conic constant between 5 and 50, and
- the posterior surface has a conic constant between -5 and -2.

This design of the Intraocular lens for cataract surgery of the invention minimizes or even eliminate the occurrence of the entoptic phenomena caused by peripheral light sources after cataract surgery.

## Description

### Field of the invention

The present invention refers to ophthalmology and in particular to an intraocular lens (IOL), in particular for cataract surgery. The invention pertains to a novel IOL design that overcomes the entoptic phenomena characterizing existing intraocular lenses.

### Background of the invention

Cataract is a medical condition where the natural lens loses its transparency, leading to a deterioration of the retinal image due to increased light scattering and aberrations. During cataract surgery, the damaged lens is removed from the eye, and a specialized implant known as an intraocular lens (IOL) is inserted to replace the refractive power of the crystalline lens. IOLs have been utilized in ophthalmology for over fifty years and have become the most common and successful procedure not only in ophthalmology but across the medical field. The chosen IOL is carefully selected to provide the necessary refractive power, optimizing focus on the retina and effectively substituting the patient's natural lens.

As a result of this implantation, the central visual field of the eye with an IOL (pseudophakic eye) is characterized by excellent image quality, primarily limited by any residual postoperative sphero-cylindrical error and, in some cases, spherical aberration, depending on the type of IOL used.

Intraocular lenses (IOLs) play a crucial role in restoring proper vision after cataract extraction. Due to the minimally invasive nature of both cataract removal and IOL implantation procedures, these lenses are designed to be small in diameter and flexible, allowing them to be folded for easier insertion.

The reduced size of these IOLs is generally adequate to cover the pupil of the eye under normal lighting conditions, particularly in older patients. However, under certain peripheral illumination, for example when night driving, the edges of the IOL may become illuminated by those peripheral light sources, leading to the emergence of various entoptic phenomena.

Entoptic phenomena associated with the limited diameter (typically 6mm) of the IOL can be grouped in two categories:
a) Positive dysphotopsias where the edge of the IOL reflects incoming light from peripheral light sources towards the central visual field, and
b) Negative dysphotopsias where part of the light is transmitted through the IOL's optic and part of the light is missing it resulting to splitting of the incoming light in two distinct peaks and leading to a dark band between them.

It must be noted that the optic of the IOL cannot be implanted arbitrarily close to the opening of the iris, as mechanical contact with the iris is to be avoided since it can be associated among others for pigment dispersion, uveitis, inflammation and increase of the intraocular pressure.

The intraocular lens is implanted in the empty lens capsule after phacoemulsification and cataract extraction. Physically the empty capsule is collapsed at a depth approximately 1mm posteriorly to the iris exposing the edge of the IOL to incoming light from light sources in the peripheral visual field.

To mitigate these phenomena, US20080269890A1 ("Intraocular Lens with Peripheral Region Designed to Reduce Negative Dysphotopsia") describes a peripheral portion near the edge of the IOL capable of diffusing the light landing at the edge of the IOL. US200802698890A1 also describes a haptic design with diffusing properties and special peripheral transition zone in the posterior surface of the lens (US20080269885A1, "IOL Peripheral Surface Designs to Reduce Negative Dysphotopsia") that will refract light from peripheral sources.

WO2020083829A1 ("Intraocular Lenses for Reducing Negative Dysphotopsia") describes an intraocular lens system that allows adjustable tilting of the IOL in order to reduce the phenomena of dysphotopsias.

WO2022189994A1 ("Intraocular Lenses for Reducing Peripheral Pseudophakic Dysphotopsia") describes a smooth control surface near the edge of the IOL.

WO2012023937A1 ("Optics and IOLs for Inhibiting Cell Migration and Reduce Optic Edge Dysphotopsia") describes a serrated edge of the IOL.

WO2023022972A1 ("Ophthalmic Prosthetic to Treat Negative and Positive Dysphotopsia") describes an optical diaphragm between the IOL and the posterior surface of the iris.

WO2016132185A1 ("Optical Implantable Member") also describes a protrusion from the peripheral portion of the lens that presumably blocks dysphotopsias.

US20080269882A1 ("Intraocular Lens with Asymmetric Optics") describes a peripheral extension to the IOL's optic.

US10561491B2 ("Reduced glare intraocular lens" describes an inclined IOL edge, similar to a chamfer.

US20220133468A1 ("Intraocular Lenses") describes a peripheral concavity near the edge of the posterior surface of the IOL, introduced at the side of the lens towards the nasal side of the eye.

WO2020083829A1 ("Intraocular Lenses for Reducing Negative Dysphotopsia") describes a posterior portion that has a peripheral change in the curvature with a sharp posterior edge.

US20080269883A1 ("Ocular Implant to Correct Dysphotopsia, Glare, Halos and Dark Shadow Type Phenomena") describes haptics with protrusions that keep the IOL very near the iris.

IOLs with inclined or textured peripheral portions concentric to the basic optical zone are also described in WO2022130141A1 ("Intraocular Lens with Rotational Resistance and Negative Dysphotopsia Mitigation") and WO2022229905A1 ("Ophthalmic Lens with Negative Dysphotopsia Mitigation & Glare Reduction").

EP3954326A1 ("Prosthetic Capsular Device") describes a structure that fills the entire capsule and therefore peripheral rays passing through the device can be diffused.

US9433498B2 ("Anti-Dysphotopic Intraocular Lens and Method") describes a lens with a peripheral notch that may engage the capsulorhexis and divert incoming peripheral rays.

Some existing solutions refer to either blocking the peripheral light that may miss the optic of the IOL or feature provisions to diffuse the light that is refracted near (or at) the edge of the IOL.

The above-mentioned inventions require special manufacturing methods and specific (non-standard) testing methods and in some cases require the implantation of additional elements (apart from the IOL).

Therefore, there is a need for Intraocular lenses that limit the phenomena of dysphotopsias while being practical to manufacture and test and surgically simple to implant.

### Summary of the invention

The object of the invention is to provide an intraocular lens for cataract surgery that overcomes the drawbacks of the prior art.

The invention presents an intraocular lens for cataract surgery comprising an anterior surface and a posterior surface, such that they form a meniscus with the anterior surface being concave and the posterior surface being convex, wherein:
- the shape factor is between -1 and -2,
- the anterior surface has a conic constant between 5 and 50, and
- the posterior surface has a conic constant between -5 and -2.

Accordingly, the present invention introduces a way for incorporating lens bending into the design of intraocular lenses. This innovative approach aims to minimize or even eliminate the occurrence of the entoptic phenomena caused by peripheral light sources.

By carefully modifying the curvature and shape of the IOL, this technique ensures that the lens edges do not interfere with peripheral light, thereby reducing the likelihood of entoptic phenomena. This advancement in IOL design promises to reduce the visual disturbances of cataract patients and enhance their visual experience.

### Brief description of the drawings

So that the matter of the invention its advantages become clear and can be understood in detail, more particular descriptions and certain embodiments of the invention briefly summarized above are illustrated in the appended drawings. These drawings form a part of the specification. It is to be noted, however, that the appended drawings illustrate preferred embodiments of the invention and therefore are not to be considered limiting in their scope.
Figure 1 shows a cross section of an eye implanted with an intraocular lens where a bundle of central rays is focused on the fovea and a bundle of peripheral rays are focused on the peripheral retina.
Figure 2a shows a peripheral ray, arriving from an angle A, being reflected at the edge of the IOL that lies a distance D behind the iris causing positive dysphotopsia.
Figure 2b shows a peripheral ray bundle arriving at the same lens, being split into two parts and causing a dark zone (DZ) between the two parts (negative dysphotopsia).
Figure 3a shows a photograph of the phenomenon described in figure 2b in an actual physical model of the human eye where the split form of the incoming light can be directly observed.
Figure 3b shows a sequence of images of a point source at different eccentricities between 60 and 75 degrees acquired at the retinal plane of a physical model of the pseudophakic eye.
Figure 4 shows three cross sections of an eye with intraocular lenses of the same power (20Diopters) but different shape factors implanted at the same depth (IOL apex at 1mm posteriorly to the iris plane) and indicative angles at which marginal rays from a 4.5 mm pupil engage the IOL's edge.
Figure 5 shows the gap between the posterior surface of the iris and the edge of the IOL (D as defined in figure 2a) as a function of the shape factor of the IOL.
Figure 6 shows the relative illumination as a function of field angle for an eye implanted with a lens with a shape factor of -1.8 (concave meniscus) and the same eye implanted with a biconvex (shape factor=0) lens.

### Detailed description of the invention

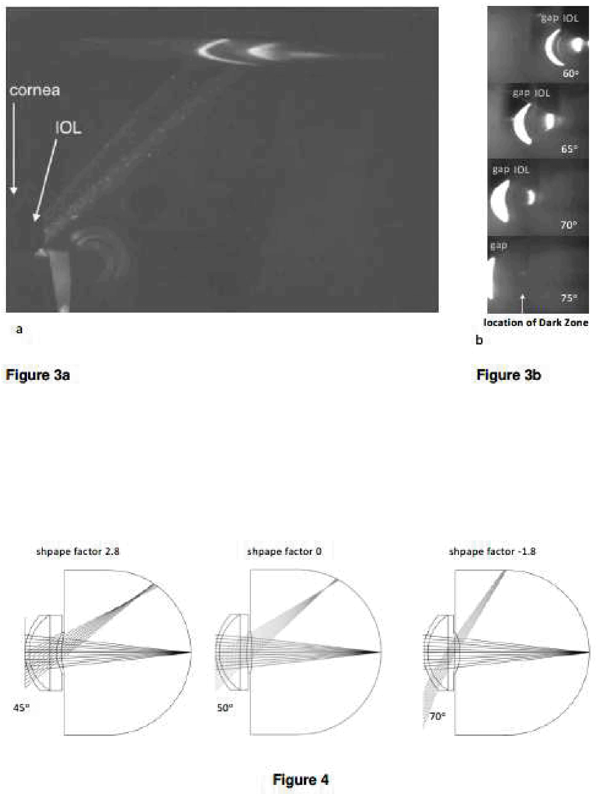

In Figure 3b there is a sequence of images of a point source at different eccentricities between 60 degrees (the uppermost sequence) and 75 degrees (the lowermost sequence) acquired at the retinal plane of a physical model of the pseudophakic eye.

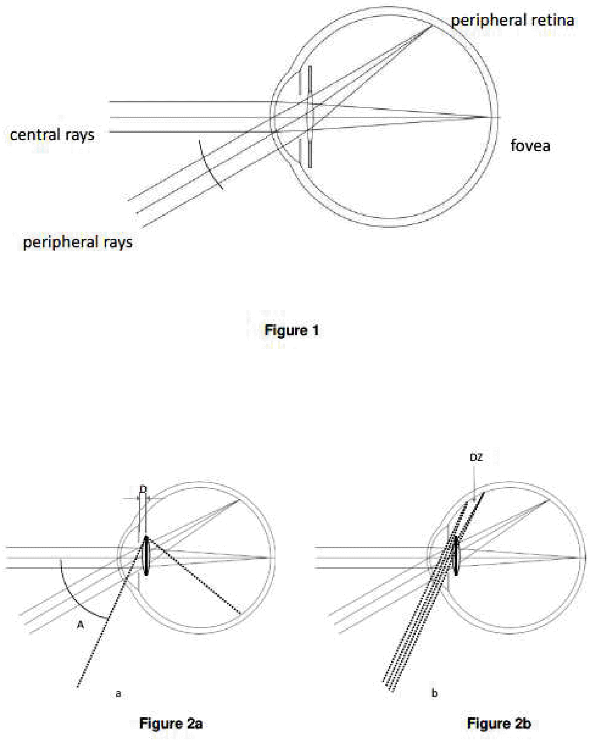

The intraocular lens is biconvex (shape factor = 0), the pupil diameter is 6 mm and the gap (distance D in Figure 2a) is 1 mm. In these conditions, at field angles greater than approximately 75 degrees, the light passing through the IOL is eliminated while the crescent persists and intensifies. This may lead to the perception of a dark band or an abrupt reduction of illuminance in the far periphery.

Figure 4 shows three cross sections of an eye with intraocular lenses of the same power (20Diopters, implemented with different shape factors), implanted at the same depth (IOL apex at 1mm posteriorly to the iris plane) and indicative angles at which marginal rays from a 4.5 mm pupil engage the IOL's edge.

These angles designate the field angles beyond which dysphotopsias may occur (45 degrees for a lens with a shape factor of 2.8; 50 degrees for a lens with a shape factor of 0; and 70 degrees for a lens with a shape factor of -1.8).

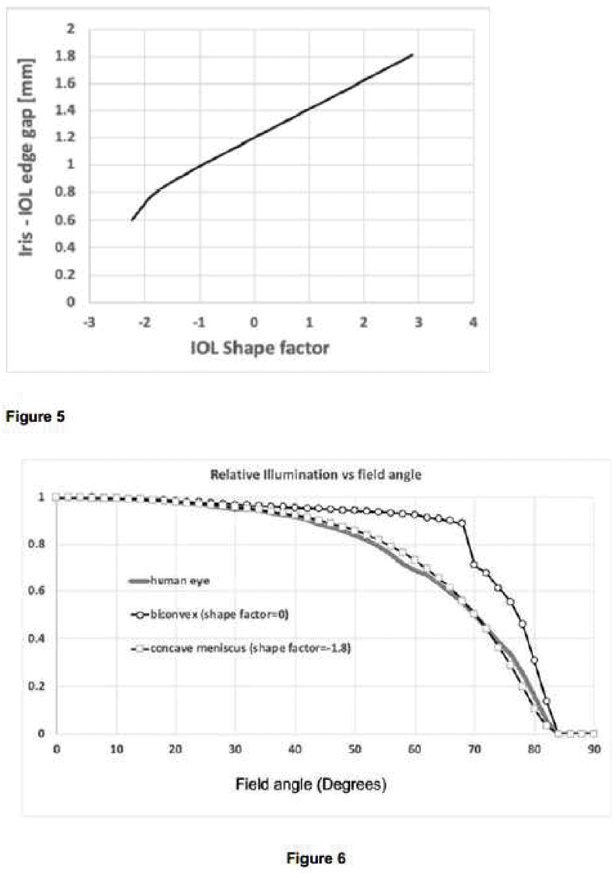

Figure 5 shows the gap between the posterior surface of the iris and the edge of the IOL (D as defined in figure 2a) as a function of the shape factor of the IOL.

The nominal depth (4.6mm) and the IOL power (20D) remain constant. It becomes obvious that negative shape factors (in this case negative shape factor designates a meniscus with its concave side towards the cornea) result to significant reduction of the gap D.

Figure 6 shows the relative illumination as a function of field angle for an eye implanted with a lens with a shape factor of -1.8 (concave meniscus) and the same eye implanted with a biconvex (shape factor =0) lens.

The relative illumination for a human eye with a model of the crystalline lens is provided for reference. The seemingly increased illuminance for the biconvex lens is associated with light missing the optical part of the IOL and landing at wider field angles as shown in figure 2b. This is an unwanted and unnatural optical performance as it is followed by an abrupt reduction of illuminance hat may perceived as a "hard stop" of the visual field. The gradual reduction of illuminance at increasing field angles is normal and it is associated with the "ellipticity" of the entrance pupil at wide field angles.

The present invention pertains to a novel posterior chamber intraocular lens (IOL) intended for implementation in cataract surgery, with a specific focus on the mitigation or elimination of dysphotopsias.

In particular, the invention refers to the application of lens bending (change of the IOL's shape factor) to design IOLs in order to position the edge of the IOL outside of the path of rays incoming from peripheral objects and in that manner reduce or eliminate dysphotopsias.

The present invention pertains to an intraocular lens for cataract surgery that is manufactured and tested using standard methods and eliminates entoptic phenomena by applying a transformation of the shape factor of the lens in order to place the IOL edge near the posterior surface of the iris while maintaining a safe spacing in the central part of the pupil.

The radii of curvature mentioned in the following sections are indicative and pertain to a high refractive index (~1.53) IOL material. For different materials the radii can be modified accordingly to achieve the designated dioptric power while maintaining the shape factor as described below.

In one preferred embodiment the IOL's shape is bent and a shape factor of about -1.8 is adopted. To achieve this, the posterior surface of the IOL has a radius of curvature between 6.5 mm and 7.5 mm, preferably of about 7mm while the anterior has a radius of curvature of about -25mm for a power of about 20 diopters. This approach positions the edge of the IOL outside of the path of rays incoming from peripheral objects and in that manner reduces or eliminates dysphotopsias. For different powers, one or both radii can be varied while maintaining the negative shape factor of the lens. Furthermore, the conic constant of the convex side is adjusted to compensate for the spherical aberration of the cornea.

In another preferred embodiment the IOL's shape is bent and a shape factor of about - 1.8 is adopted. To achieve this, the posterior surface of the IOL has a radius of curvature between 6.5 mm and 7.5 mm, preferably of about 7mm while the anterior has a radius of curvature of about -25mm for a power of about 20 diopters. This approach positions the edge of the IOL outside of the path of rays incoming from peripheral objects and in that manner reduces or eliminates dysphotopsias. For different powers, one or both radii can be varied while maintaining the negative shape factor of the lens. Furthermore, the conic constant of both surfaces is adjusted in a balanced manner to compensate for the spherical aberration of the cornea. In this embodiment the posterior surface may have a conic constant ranging from about -5 to about -2 and the anterior surface may have a conic constant ranging from about 5 to about 50 for the most common powers.

In another preferred embodiment the IOL's shape is bent and a shape factor of about - 1.8 is adopted. To achieve this, the posterior surface of the IOL has a radius of curvature between 6.5 mm and 7.5 mm, preferably of about 7mm while the anterior has a radius of curvature of about -25mm for a power of about 20 diopters. This approach positions the edge of the IOL outside of the path of rays incoming from peripheral objects and in that manner reduces or eliminates dysphotopsias. For different powers, one or both radii can be varied while maintaining the negative shape factor of the lens. Furthermore, the conic constant of both surfaces is adjusted so that the anterior (concave) surface has a substantial amount of negative spherical aberration, achieved by a positive conic constant (for example ranging from about 30 to about 60) in order to further increase the curvature in the periphery towards the iris and further reduce the gap between the edge of the IOL and the iris. The posterior surface is then adjusted to achieve the required spherical aberration, either to compensate for the spherical aberration of the cornea or to introduce any other deliberate amount of spherical aberration.

In another preferred embodiment the IOL's shape is bent and a shape factor of about - 1.8 is adopted. To achieve this the posterior surface of the IOL has a radius of curvature between 6.5 mm and 7.5 mm, preferably of about 7mm while the anterior has a radius of curvature of about -25mm for a power of about 20 diopters. This approach positions the edge of the IOL outside of the path of rays incoming from peripheral objects and in that manner reduces or eliminates dysphotopsias. For different powers, one or both radii can be varied while maintaining the negative shape factor of the lens. Furthermore, the conic constant of both surfaces is adjusted in a balanced manner to introduce a given amount of negative spherical aberration ranging from about -0.3 to about -0.7 micrometers for an entrance pupil of 6mm as specified in ISO 11979-2 (ISO model eye 2).

In another preferred embodiment the IOL's shape is bent and a shape factor of about - 1.2 is adopted. To achieve this the posterior surface of the IOL has a radius of curvature of about 9mm while the anterior has a radius of curvature of about -100mm for a power of about 20 diopters. This approach positions the edge of the IOL outside of the path of rays incoming from peripheral objects and in that manner reduces or eliminates dysphotopsias. For different powers, one or both radii can be varied while maintaining the overall negative shape factor of the lens. Furthermore, the conic constant of both surfaces is adjusted in a balanced manner to compensate for the spherical aberration of the cornea. In this embodiment the posterior surface may have a conic constant ranging from about -5 to about -2 and the anterior surface may have a conic constant ranging from about 5 to about 50 for the most common powers.

In another preferred embodiment the IOL's shape is bent with the shape factor ranging between -1 and -2. In addition to bending the shape of the IOL an increase of the IOL's diameter may further reduce the gap between the IOL's edge and the posterior iris (provided that the shape factor is negative). In this embodiment the IOL has a negative shape factor smaller than -1 and ranging between -1.2 and -1.8 and its diameter is increased to 6.5 mm compared to the "industry standard" 6mm. Furthermore, the conic constant of both surfaces is adjusted in a balanced manner to compensate for the spherical aberration of the cornea. In this embodiment the posterior surface may have a conic constant ranging from about -5 to about -2 and the anterior surface may have a conic constant ranging from about 5 to about 50 for the most common powers.

The value of about 7 mm for the radius of curvature of the posterior surface (and in particular the range of values between 6.5 and 7.5 mm) is not a random selection equivalent to other similar values. It has been carefully chosen following a design process in which the following points have been taken into account:
- This curvature provides substantial lens bending to effectively completely close the gap between the posterior iris and the edge of the lens. A flatter curvature (e.g. 9 mm) would still allow a 0.3 mm (typical) gap.
- This curvature if maintained as a constant for all IOL powers will result to a gap closure independent of IOL power. In the present invention the posterior curvature is kept constant across IOL powers to ensure gap closure at all IOL powers. This could not have been achieved with an even steeper curvature as this would substantially increase the sagittal height between the vertex of the posterior surface and the IOL rim to an extent that the IOL could press against the posterior capsule and the posterior side of the iris.
- The chosen value is a result of the understanding of optics, IOL design, negative dysphotopsia, biomechanics of the eye and surgical requirements and represents a best design as it reflects a best balance between manufacturing tolerances (steep curvatures are more difficult to manufacture precisely) and gap closure effectiveness.
- Said value allows the combination with different anterior surfaces to create a variety of IOL powers without the need of extreme curvatures for the anterior surface. The fact a single posterior surface is used for all the powers is unique to this design, it serves the purpose of gap closure to prevent dysphotopsia and also promotes precision as there is only one "difficult" surface across the power range that the manufacturer can optimise (e.g. in a molding process).

## Claims

1. Intraocular lens for cataract surgery, comprising an anterior surface and a posterior surface, such that they form a meniscus with the anterior surface being concave and the posterior surface being convex, wherein:
- the shape factor is between -1 and -2,
- the anterior surface has a conic constant between 5 and 50, and
- the posterior surface has a conic constant between -5 and -2.

2. Intraocular lens for cataract surgery, according to claim 1, wherein the shape factor is between -1.2 and -1.8.

3. Intraocular lens for cataract surgery, according to claim 2, wherein:
- the shape factor is -1.8,
- the anterior surface has a radius of curvature of -25 mm, and
- the posterior surface has a radius of curvature between 6.5 mm and 7.5 mm.

4. Intraocular lens for cataract surgery, according to claim 3, wherein the posterior surface has a radius of curvature of 7 mm.

5. Intraocular lens for cataract surgery, according to claim 2, wherein:
- the shape factor is -1.2,
- the anterior surface has a radius of curvature of -100 mm, and
- the posterior surface has a radius of curvature of 9 mm.

6. Intraocular lens for cataract surgery, according to claim 3, 4 or 5, the intraocular lens having a Power of 20D.

7. Intraocular lens for cataract surgery, according to any of the previous claims, wherein the diameter of the lens is 6.5 mm.
